# EUROPEAN PATENT SPECIFICATION

(11) **EP 2 027 277 B1**
(45) Date of publication and mention of the grant of the patent: **18.07.2018**
(21) Application number: 07729987.3
(22) Date of filing: 07.06.2007
(51) Int. Cl.: C12P 7/42

(54) **GLYCOLIC ACID PRODUCTION BY FERMENTATION FROM RENEWABLE RESOURCES**
GLYCOLSÄUREHERSTELLUNG DURCH FERMENTIERUNG AUS ERNEUERBAREN RESSOURCEN
PRODUCTION D'ACIDE GLYCOLIQUE PAR FERMENTATION À PARTIR DE RESSOURCES RENOUVELABLES

(30) Priority: 09.06.2006 WO PCT/EP2006/063046
(43) Date of publication of application: 25.02.2009
(73) Proprietor: Metabolic Explorer, 63360 Saint Beauzire (FR)
(72) Inventor: SOUCAILLE, Philippe, 31450 Deyme (FR)
(74) Representative: Regimbeau
(86) International application number: PCT/EP2007/055625
(87) International publication number: WO 2007/141316

(56) References cited:
- EP-A1- 1 748 076
- WO-A-02/068658
- DATABASE WPI Week 199715 Derwent Publications Ltd., London, GB; AN 1997-159097 XP002420145 & JP 09 028390 A (NITTO CHEM IND CO LTD) 4 February 1997 (1997-02-04)
- NUNEZ M FELISA ET AL: "Biochemical characterization of the 2-ketoacid reductases encoded by ycdW and yiaE genes in Escherichia coli" BIOCHEMICAL JOURNAL, vol. 354, no. 3, 15 March 2001 (2001-03-15), pages 707-715, XP002420142 ISSN: 0264-6021 cited in the application
- YUN HYUNGDON ET AL: "Stereospecific synthesis of (R)-2-hydroxy carboxylic acids using recombinant E-coli BL21 overexpressing YiaE from Escherichia coli K12 and glucose dehydrogenase from Bacillus subtilis" BIOTECHNOLOGY PROGRESS, vol. 21, no. 2, March 2005 (2005-03), pages 366-371, XP002420143 ISSN: 8756-7938

## Description

### FIELD OF INVENTION

The invention comprises a process for the bioconversion of a fermentable carbon source to glycolic acid by an aerobically-grown microorganism.

### BACKGROUND OF THE INVENTION

Glycolic acid (HOCH₂COOH) is the first member of the alpha-hydroxy acid family of carboxylic acids. Glycolic acid has dual functionality with both alcohol and moderately strong acid functional groups on a very small molecule. This results in unique chemical attributes as well as typical acid and alcohol chemistry.

Glycolic acid uses both the hydroxyl and carboxylic acid groups to form five-member ring complexes (chelates) with polyvalent metals. This metal ion complexing ability is useful in dissolution of hard water scale and prevention of deposition, especially in acid cleaning applications where good rinsibility is a key factor. Glycolic acid undergoes reactions with organic alcohols and acids to form esters. Low molecular weight alkyl glycolic esters have unusual solvency properties and may be used as a substitute for n- and iso-propanol, ethylenediamine, phenol, m-cresol, 2-ethoxyethyl acetate, and ethyl and methyl lactate. Higher molecular weight alkyl esters can be used in personal care product formulations. Glycolic acid can react with itself to form dimeric glycolide, head-to-tail polyester oligomers, and long-chain polymers. Copolymers can be made with other alpha hydroxy acids like lactic acid. The polyester polymers gradually hydrolyze in aqueous environments at controllable rates. This property makes them useful in biomedical applications such as dissolvable sutures and in applications where a controlled release of acid is needed to reduce pH. Currently more than 15,000 tons of glycolic acid are consumed annually in the United states.

Methods for enzymatic conversion of a substrate into glycolic acid are disclosed in the art. All these methods comprise preparing an enzyme by culturing a microorganism and then using the enzyme being produced, possibly with the whole microorganism cell, for converting a complex substrate in a reaction medium. The complex substrate may be glycolonitrile, converted into glycolate by a nitrilase (WO 02/068658; JP 09 028390). The complex substrate may be a 2-oxo-carboxylic acid converted stereospecifically into the corresponding (R)-2-hydroxy carboxylic acid with the combination of two enzymes, YiaE and a glucose dehydrogenase (Yun & al., Biotechnology Progress, vol. 21, no. 2, 2005, 366-371). The substrate may be ethylene glycol, converted into glycolate by lactaldehyde reductase and lactaldehyde dehydrogenase (WO-A-2005106005). The biological production of glycolic acid, presented in figure 1, requires the formation of glyoxylate as an intermediate which is reduced to glycolate by a NADPH dependent oxidoreductase encoded by the gene *ycd*W (Nunez et al, (2001) Biochemistry, 354, 707-715). Glyoxylate is an intermediate of the glyoxylate cycle (Tricarboxylic acid cycle and glyoxylate bypass, reviewed in Neidhardt, F. C. (Ed. in Chief), R. Curtiss III, J. L. Ingraham, E. C. C. Lin, K. B. Low, B. Magasanik, W. S. Reznikoff, M. Riley, M. Schaechter, and H. E. Umbarger (eds). 1996. Escherichia coli and Salmonella: Cellular and Molecular Biology. American Society for Microbiology). In this cycle isocitrate is cleaved into succinate and glyoxylate, a reaction that is catalyzed by isocitrate lyase, encoded by the *ace*A gene. Succinate directly enters the citric acid cycle and is converted into oxaloacetate. Glyoxylate is converted into malate by incorporating a molecule of acetyl-CoA derived from acetate a reaction catalyzed by the two malate synthase isoenzymes encoded by *ace*B and *gclB.* The entry of carbon into the glyoxylate shunt is regulated on the transcriptional and posttranscriptional level. Transcriptional regulation is exerted on the *ace*BAK operon by the IclR repressor. AceBAK encode malate synthase, isocitrate lyase and isocitrate kinase/phosphatase, respectively. The *icl*R gene is negatively autoregulated and activated by the FadR protein. The activity of isocitrate dehydrogenase, encoded by the *icd* gene, is regulated posttranscriptionally. Isocitrate dehydrogenase and isocitrate lyase compete for the common substrate isocitrate. Since the Kₘ value for isocitrate is significantly higher for the isocitrate lyase reaction, the entry into the glyoxylate pathway depends in part on the regulation of the isocitrate dehydrogenase enzyme. Isocitrate dehydrogenase activity is modulated by its phosphorylation or dephosphorylation catalyzed by AceK. Phosphorylation reduces the activity of led and dephosphorylation reactivates the led enzyme. If AceK acts as kinase or phosphatase depends on the presence of several metabolites. Depletion of isocitrate and 3-phosphoglycerate stimulates kinase activity; the presence of pyruvate and AMP inhibits the kinase function thus favoring the phosphatase activity (see also Neidhard). Glyoxylate can be converted to tartronate semialdehyde by a glyoxylate carboligase encoded by *gcl* and to 2-keto-4-hydroxy glutarate by a 2-keto-3-deoxygluconate 6-phosphate aldolase encoded by *eda* while glycolate can be reduced to glycoaldehyde by a NAD⁺ dependent glycoaldehyde dehydrogenase encoded by *aldA* or oxidized to glyoxylate by a NAD⁺ dependent glycolate oxidase encoded by *glc*DEF.

The problem to be solved by the present invention is the biological production of glycolic acid from an inexpensive carbon substrate such as glucose or other sugars. The number of biochemical steps and the complexity of the metabolic pathways necessitate, for an industrial feasible process of glycolic acid production, the use of a metabolically engineered whole cell catalyst.

### SUMMARY OF THE INVENTION

Applicants have solved the stated problem and the present invention provides a method for bioconverting a fermentable carbon source capable of being metabolized by a recombinant microorganism modified for directly converting said source of carbon to glycolic acid. The microorganism is modified to attenuate the conversion of glyoxylate to products other than glycolate, with the attenuation of the expression of at least one gene, selected among the following genes involved in glyoxylate metabolism:
aceB encoding malate synthase,
glcB encoding the second malate synthase,
gcl encoding glyoxylate carboligase,
eda encoding 2-keto-3-deoxygluconate 6-phosphate aldolase.
The fermentable source of carbon is a sugar selected among monosaccharides, disaccharides, trisaccharides, oligosaccharides and polysaccharides. The method of the invention comprises the following steps:
a) Fermentation of the microorganism to produce glycolic acid by converting the source of carbon into glycolic acid,
b) Concentration of the glycolic acid in the bacteria or in the medium and
c) Recovery of glycolic acid from the fermentation broth and/or the biomass optionally remaining in portions or in the total amount (0-100%) in the end product. Glucose is used as a model substrate and recombinant *E. coli* is used as the model host. In one aspect of this invention, recombinant *E. coli* unable to metabolize glyoxylate to other compounds than glycolate are constructed by inactivating the genes *aceB' glcB' gcl* and *eda*. In another aspect of this invention, an NADPH dependant glyoxylate reductase activity is used to reduce the toxic glyoxylate into glycolate by using endogenous encoding genes like *ycd*W or *yia*E*.* In a further aspect of this invention the gene encoding the glycolate metabolizing enzymes, glycolate oxidase (*glc*DEF) and glycoaldehyde dehydrogenase (*aldA*) are deleted. Futhermore, the flux in the glyoxylate pathway is increased by i) increasing the level of aceA by inactivating the *iclR* gene or directly increasing the expression of *aceA*, ii) decreasing the expression level or inactivating the gene encoding the isocitrate dehydrogenase (*icd*) and iii) inactivating the genes encoding the pyruvate oxidase (*pox*B) and the acetate pathway (*ack*, *pta*)*.* In a final aspect of this invention, a better yield of glycolate production is obtained by increasing NADPH availability by inactivating the genes encoding the glucose-6-phosphate isomerase (*pgi*), the 6-phosphogluconate dehydratase (*edd*) and the soluble transhydrogenase (*udh*A). The present invention may be generally applied to include any carbon substrate that is readily converted to acetyl-coA.

Accordingly it is an object of the present invention to provide a recombinant organism, useful for the production of glycolic acid comprising: (a) at least inactivation of all the malate synthases, glyoxylate carboligases and 2-keto-3-deoxygluconate 6-phosphate aldolase encoding genes; (b) at least one gene encoding a polypeptide having NADPH dependent glyoxylate reductase activity and (c) at least inactivation of the genes encoding NAD⁺ dependant glycolate oxidation to glyoxylate. Optionally the recombinant organism may comprise i) inactivating mutations in endogenous genes selected from the group consisting of: (a) a gene encoding a repressor of the glyoxylate pathway (b) a gene encoding a polypeptide having glucose-6-phosphate isomerase activity. (c) a gene encoding a polypeptide having soluble transhydrogenase activity. (d) a gene encoding a polypeptide having 6-phosphogluconate dehydratase activity (e) genes encoding polypeptides having phospho-transacetylase and acetate kinase activities. (f) a gene encoding pyruvate oxidase activity (g) a gene encoding glycoaldehyde dehydrogenase activity ii) increase level of a gene encoding isocitrate lyase and iii) decrease level or inactivation of a gene encoding polypeptide having isocitrate dehydrogenase activity.

In another embodiment the invention provides a process for the production of glycolic acid from a recombinant organism comprising: (a) contacting the recombinant organism of the present invention with at least one carbon source selected from the group consisting of monosaccharides, oligosaccharides, polysaccharides, and single-carbon substrates whereby glycolate is produced; optionally (b) recovering the glycolic acid produced in (a) through a step of polymerization to at least glycolic acid dimers and (c) recovery of glycolic acid by depolymerisation from glycolic acid dimmers, oligomers and/or polymers.

### BRIEF DESCRIPTION OF THE DRAWINGS

The accompanying drawings which are incorporated in and constitute a part of this specification exemplify the invention and together with the description, serve to explain the principles of this invention.

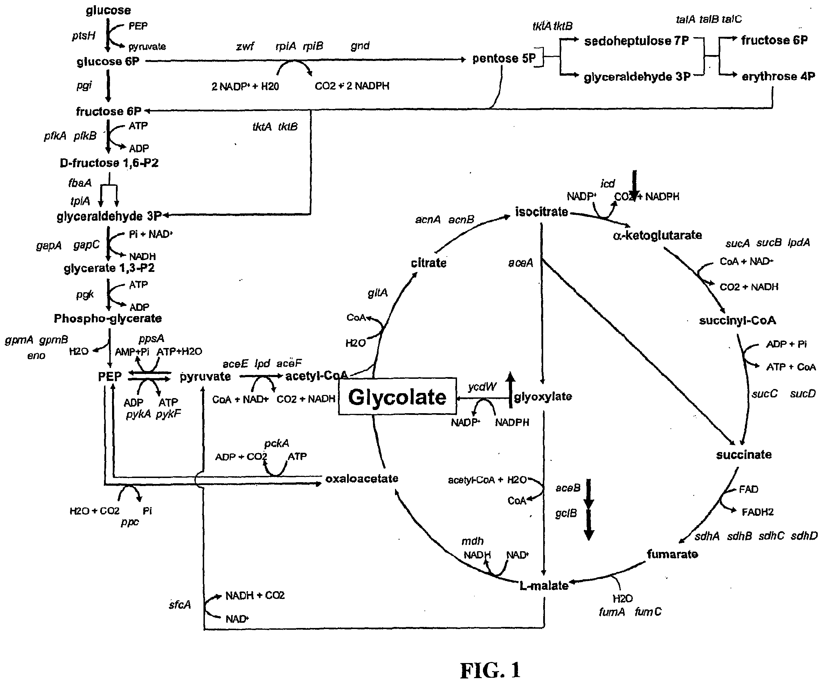

**Figure 1** depicts the genetic engineering of glycolysis, TCA cycle and glyoxylate pathway in the development of glycolic acid production system from carbohydrates.

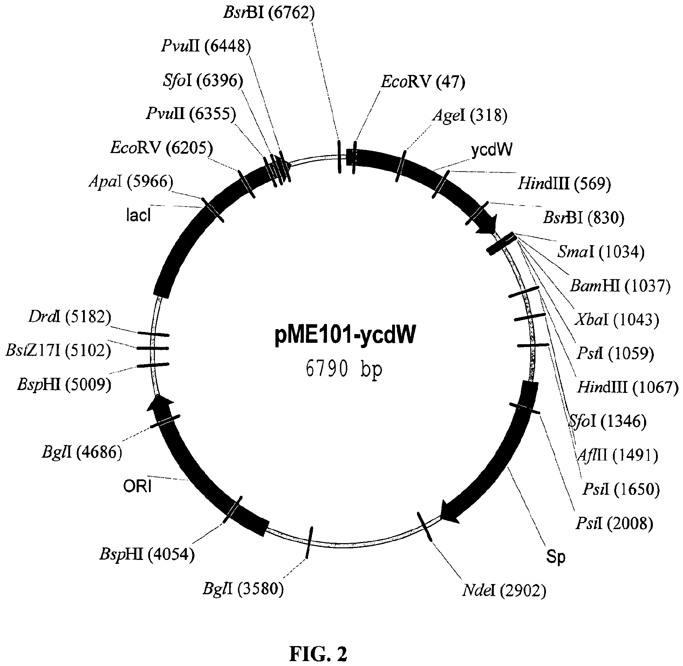

**Figure 2** is a diagram showing the construction of the vector pME101- ycdW.

### DETAILED DESCRIPTION OF THE INVENTION

As used herein the following terms may be used for interpretation of the claims and specification.
The term "mutant strain" refers to a non-wild type strain.
The term "microorganism" refers to all kind of unicellular organisms, including procaryotic organisms like bacteria, and eucaryotic organisms like yeasts. Bacteria include in particular : Enterobacteriaceae, Bacillaceae, Streptomycetaceae and Corynebacteriaceae. Enterobacteriaceae comprise in particular but not exclusively the genera Escherichia, Klebsiella, Salmonella and Pantoea.

The term "transformation" or "transfection" refers to the acquisition of new genes in a cell after the incorporation of exogenous nucleic acid. The term "transformant" refers to the product of a transformation. The term "genetically altered" refers to the process of changing hereditary material by transformation or mutation.
The term "attenuation" refers to a decreased expression of a gene or a decreased activity of the protein, product of the gene. The man skilled in the art knows numerous means to obtain this result, and for example:
- Introduction of a mutation into the gene, decreasing the expression level of this gene, or the level of activity of the encoded protein.
- Replacement of the natural promoter of the gene by a low strength promoter, resulting in a lower expression.
- Use of elements destabilizing the corresponding messenger RNA or the protein
- Deletion of the gene if no expression is needed.
The term "expression" refers to the transcription and translation from a gene to the protein, product of the gene.

The term "plasmid" or "vector" as used herein refers to an extra chromosomal element often carrying genes which are not part of the central metabolism of the cell, and usually in the form of circular double-stranded DNA molecules.

The term "carbon substrate" or "carbon source" means any carbon source capable of being metabolized by a microorganism wherein the substrate contains at least one carbon atom. Authors refer particularly to renewable, inexpensive and fermentable carbon sources such as monosaccharides, oligosaccharides, polysaccharides, single-carbon substrates, and polyols such as glycerol. Single carbon substrate are defined as carbon molecules that contain only one carbon atom such as methanol. Monosaccharides of the formula (CH₂O)ₙ are also called oses or "simple sugars"; monosaccharides include saccharose, fructose, glucose, galactose and mannose. Other carbon sources comprising more than one monosaccharide are called disaccharides, trisaccharides, oligosaccharides and polysaccharides. Disaccharides include saccharose (sucrose), lactose and maltose. Starch and hemicellulose are polysaccharides, also known as "complex sugars". Therefore the term "carbon source" means any product as cited above, and mixtures thereof.

The term "ATCC" will stand for the American Type Culture Collection, 12301 Parklawn Drive, Rockville, Md. 20852, U.S.A.
The terms "glyoxylate" and "glyoxylic acid" are used interchangeably.
The terms "glycolate" and "glycolic acid" are used interchangeably.

In the description of the present invention, enzymes are identified by their specific activities. This definition thus includes all polypeptides that have the defined specific activity also present in other organisms, more particularly in other microorganisms. Often enzymes with similar activities can be identified by their grouping to certain families defined as PFAM or COG.

PFAM (protein families database of alignments and hidden Markov models; http://www.sanger.ac.uk/Software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/) are obtained by comparing protein sequences from 43 fully sequenced genomes representing 30 major phylogenic lines. Each COG is defined from at least three lines, which permits the identification of former conserved domains.

The means of identifying homologous sequences and their percentage homologies are well known to those skilled in the art, and include in particular the BLAST programs, which can be used from the website http://www.ncbi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://prodes.toulouse.inra.fr/multalin/cgi-bin/multalin.pl), with the default parameters indicated on those websites.

Using the references given on GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, for example, in Sambrook et al. (1989 Molecular Cloning: a Laboratory Manual. 2nd ed. Cold Spring Harbor Lab., Cold Spring Harbor, New York.).
The present invention provides a method for the fermentative production of glycolic acid, its derivatives or precursors, by culturing a microorganism in an appropriate culture medium comprising a carbon source and the recovery of glycolic acid from the culture medium.

A further embodiment of the invention provides a method wherein the microorganism is modified to have a low capacity of glyoxylate conversion, except to produce glycolate, due to the attenuation of genes encoding for enzymes consuming glyoxylate, a key precursor of glycolate: *ace*B and *gclB genes encoding* malate synthases, *gcl* encoding glyoxylate carboligase and *eda* encoding 2-keto-3-deoxygluconate 6-phosphate aldolase.

In another embodiment of the invention, the microorganism contains at least one gene encoding a polypeptide catalyzing the conversion of glyoxylate to glycolate.

In particular, a gene encoding a NADPH dependent glyoxylate reductase enzyme is present to convert, under aerobic conditions, the toxic glyoxylate intermediate to the low toxicity final product glycolate. The gene can be exogenous or endogenous and can be expressed chromosomally or extrachromosomally. An NADPH-dependant glyoxylate reductase encoding gene can be taken among the *ycd*W or *yia*E genes from the genome of *E. coli* MG1655. In a preferred embodiment, the expression of at least one of said genes is increased. If needed a high level of NADPH-dependant glyoxylate reductase activity can be obtained from chromosomally located genes by using one or several copies on the genome that can be introduced by methods of recombination known to the expert in the field. For extrachromosomal genes, different types of plasmids that differ with respect to their origin of replication and thus their copy number in the cell can be used. They may be present as 1-5 copies, ca 20 or up to 500 copies corresponding to low copy number plasmids with tight replication (pSC101, RK2), low copy number plasmids (pACYC, pRSF1010) or high copy number plasmids (pSK bluescript II). The *ycd*W or *yia*E genes may be expressed using promoters with different strength that need or need not to be induced by inducer molecules. Examples are the promoters Ptrc, Ptac, Plac, the lambda promoter cI or other promoters known to the expert in the field. Expression of the genes may also be boosted by elements stabilizing the corresponding messenger RNA (Carrier and Keasling (1998) Biotechnol. Prog. 15, 58-64) or the protein (e.g. GST tags, Amersham Biosciences).

In a further embodiment of the invention, the microorganism is modified in such a way that it is unable to substantially metabolize glycolate. This result can be achieved by the attenuation of at least one of the genes encoding for enzymes consuming glycolate (*glcDEF* encoding glycolate oxidase and *aldA* encoding glycoaldehyde dehydrogenase). Attenuation of genes can be done by replacing the natural promoter by a low strength promoter or by element destabilizing the corresponding messenger RNA or the protein. If needed, complete attenuation of the gene can also be achieved by a deletion of the corresponding DNA sequence.
In another embodiment, the microorganism used in the method of the invention is transformed to increase the glyoxylate pathway flux.
The flux in the glyoxylate pathway may be increased by different means, and in particular:
i) decreasing the activity of the enzyme isocitrate dehydrogenase (Icd),
ii) decreasing the activity of at least one of the following enzymes:
   - phospho-transacetylase, encoded by the *pta* gene
   - acetate kinase, encoded by the *ack* gene
   - pyruvate oxidase, encoded by the *pox*B gene by attenuation of the genes,
iii) increasing the activity of the enzyme isocitrate lyase, encoded by the *aceA* gene.
Decreasing the level of isocitrate dehydrogenase can be accomplished by introducing artificial promoters that drive the expression of the *icd* gene, coding for the isocitrate dehydrogenase, or by introducing mutations into the *icd* gene that reduce the enzymatic activity of the protein.
Since the activity of the protein Icd is reduced by phosphorylation, it may also be controlled by introducing mutant *ace*K genes that have increased kinase activity or reduced phosphatase activity compared to the wild type AceK enzyme.
Increasing the activity of the isocitrate lyase can be accomplished either by attenuating the level of *iclR or fadR genes,* coding for glyoxylate pathway repressors, either by stimulating the expression of the *aceA* gene, for example by introducing artificial promoters that drive the expression of the gene, or by introducing mutations into the *aceA* gene that increase the activity the encoded protein.

An embodiment of the invention provides a better yield of glycolate production by increasing NADPH availability to the NADPH-dependant glyoxylate reductase. This modification of the microorganism characteristics can be obtained through the attenuation of at least one of the genes selected among the following : *pgi* encoding the glucose-6-phosphate isomerase, *udhA* encoding the soluble transhydrogenase and *edd* encoding the 6-phosphogluconate dehydratase activity. With such genetic modifications, all the glucose-6-phosphate will have to enter glycolysis through the pentose phosphate pathway and 2 NADPH will be produced per glucose-6-phosphate metabolized.

In another embodiment the invention provides a process for the fermentative production of glycolic acid from a recombinant organism comprising: (a) contacting the recombinant organism of the present invention with at least one carbon source selected from the group consisting of glucose, sucrose, monosaccharides, oligosaccharides, polysaccharides, starch or its derivatives, glycerol and single-carbon substrates whereby glyoxylic acid is produced. Optionaly the process comprises a step of concentration of glycolate in the bacteria or in the medium and isolation of glycolic acid from the fermentation broth and/or the biomass optionally remaining in portions or in the total amount (0-100%) in the end product. Optionally the process comprises a step of recovery of the glycolic acid produced in step (a) through a step of polymerization to at least glycolic acid dimers and (b) recovery of glycolic acid by depolymerisation from glycolic acid dimers, oligomers and/or polymers.
Those skilled in the art are able to define the culture conditions for the microorganisms according to the invention. In particular the bacteria are fermented at a temperature between 20°C and 55°C, preferentially between 25°C and 40°C, and more specifically about 30°C for C. *glutamicum* and about 37°C for *E*. *coli.*
The fermentation is generally conducted in fermenters with an inorganic culture medium of known defined composition adapted to the bacteria used, containing at least one simple carbon source, and if necessary a co-substrate necessary for the production of the metabolite.

The invention is also related to the microorganism as described previously. Preferably, this microorganism is selected among the group consisting of *E*. *coli, C. glutamicum* or *S. cerevisiae.*

### EXAMPLE 1

### Construction of strains unable to metabolize glyoxylate except to reduce it to glycolate: MG1655 ΔaceB Δgcl ΔglcB

To delete the *ace*B gene the homologous recombination strategy described by Datsenko & Wanner (2000) is used. This strategy allows the insertion of a chloramphenicol or a kanamycin resistance cassette, while deleting most of the genes concerned. For this purpose the following oligonucleotides are used:
DaceBF (SEQ ID NO 1) 
ggcaacaacaaccgatgaactggctttcacaaggccgtatggcgagcaggagaagcaaattcttactgccgaagcggtagCA
TATGAATATCCTCCTTAG
 with
   - a region (lower case) homologous to the sequence (4213068-4213147) of the gene *ace*B (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),
DaceBR (SEQ ID NO 2)) 
ggcggtagcctggcagggtcaggaaatcaattaactcatcggaagtggtgatctgttccatcaagcgtgcggcatcgtcTGTAG
GCTGGAGCTGCTTCG
 with
- a region (lower case) homologous to the sequence (4214647-4214569) of the gene *ace*B (reference sequence on the website http://genolist.pasteur.fr/Colibri/)*,*
- a region (upper case) for the amplification of the chloramphenicol resistance cassette (reference sequence in Datsenko, K.A. & Wanner, B.L., 2000, PNAS, 97: 6640-6645).
The oligonucleotides DaceBF and DaceBR are used to amplify the chloramphenicol resistance cassette from the plasmid pKD3. The PCR product obtained is then introduced by electroporation into the strain MG1655 (pKD46), in which the Red recombinase enzyme expressed permits the homologous recombination. The chloramphenicol resistant transformants are then selected and the insertion of the resistance cassette is verified by a PCR analysis with the oligonucleotides aceBF and aceBR defined below. The strain retained is designated MG1655 Δ*ace*B::Cm
aceBF (SEQ ID NO 3) : cgttaagcgattcagcaccttacc (homologous to the sequence from 4212807 to 4212830).
aceBR (SEQ ID NO 4) : ccagtttctgaatagcttcc (homologous to the sequence from 4215327 to 4215308).

Then, the *gcl* gene is deleted in the MG1655 Δ*ace*B::Cm strain by transduction. The MG1655 Δ*gcl*::Km strain is first constructed using the same method as previously described with the following oligonucleotides :
DgclF (SEQ ID NO 5) 
ggcaaaaatgagagccgttgacgcggcaatgtatgtgctggagaaagaaggtatcactaccgccttcggtgttccgggagcTG
TAGGCTGGAGCTGCTTCG
 with
   - a region (lower case) homologous to the sequence (533142-533224) of the region of the gene *gcl* (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DgipR (SEQ ID NO 6) 
gcgttacgttttaacggtacggatccatccagcgtaaaccggcttccgtggtggtttggggtttatattcacacccaacccCATAT
GAATATCCTCCTTAG
 with
   - a region (lower case) homologous to the sequence (535720-535640) of the region of the gene *gcl* (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645).

The oligonucleotides DgclF and DgipR are used to amplify the kanamycin resistance cassette from the plasmid pKD4. The PCR product obtained is then introduced by electroporation into the strain MG1655 (pKD46). The kanamycin resistant transformants are then selected and the insertion of the resistance cassette is verified by a PCR analysis with the oligonucleotides gclF and gipR defined below. The strain retained is designated MG1655 Δ*gcl*::Km.
gclF (SEQ ID NO 7) : ggatatgcccaccttgctgaagg (homologous to the sequence from 532795 to 532817).
gipR (SEQ ID NO 8) : cgcttagtttcaatcggggaaatgg (homologous to the sequence from 536114 to 536090).
To transfer the deletion Δ*gcl*::Km, the method of phage PI transduction is used. The protocol followed is implemented in 2 steps with the preparation of the phage lysate of the strain MG1655 Δ*gcl*::Km and then transduction into strain MG1655 Δ*ace*B::Cm. The construction of the strain is described above.

### Preparation of phage lysate PI:

- Inoculation with 100 µl of an overnight culture of the strain MG1655 Δ*gcl*::Km of 10 ml of LB + Km 50 µg/ml + glucose 0.2% + CaCl₂ 5 mM.
- Incubation for 30 min at 37°C with shaking.
- Addition of 100 µl of phage lysate PI prepared on the strain MG1655 (about 1.10⁹ phage/ml).
- Shaking at 37°C for 3 hours until all the cells were lysed.
- Addition of 200 µl chloroform and vortexing.
- Centrifugation for 10 min at 4500 g to eliminate cell debris.
- Transfer of supernatant to a sterile tube and addition of 200 µl chloroform.
- Storage of lysate at 4°C.

### Transduction

- Centrifuging for 10 min at 1500 g of 5 ml of an overnight culture of the strain MG1655 Δ*ace*B::Cm in LB medium.
- Suspension of the cell pellet in 2.5 ml of 10 mM MgSO₄, 5 mM CaCl₂
- Control tubes: 100 µl cells
   100 µl phages PI of strain MG1655 Δ*gcl*::Km
- Test tube: 100 µl of cells + 100 µl of phages PI of the strain MG1655 Δ*gcl*::Km.
- Incubation for 30 min at 30°C without shaking.
- Addition of 100 µl of 1 M sodium citrate in each tube and vortexing.
- Addition of 1 ml of LB.
- Incubation for 1 hour at 37°C with shaking.
- Spreading on dishes LB + Km 50 µg/ml after centrifuging of tubes for 3 min at 7000 rpm.
- Incubation at 37°C overnight.

### Verification of the strain

The kanamycin resistant transformants are then selected and the deletion of the gene Δ*gcl*::Km is verified by a PCR analysis with the oligonucleotides gclF and gipR previously described. The strain retained is designated MG1655 Δ*ace*B::Cm Δ*gcl*::Km.

The kanamycin and chloramphenicol resistance cassettes can then be eliminated. The plasmid pCP20 carrying FLP recombinase acting at the FRT sites of the kanamycin and the chloramphenicol resistance cassettes is then introduced into the recombinant sites by electroporation. After a series of cultures at 42°C, the loss of the kanamycin and chloramphenicol resistance cassettes is verified by a PCR analysis with the same oligonucleotides as used previously (aceBF / aceBR and gclF / gipR). The strain retained is designated MG1655 Δ*ace*B Δ*gcl.*

Then, the *glc*B gene is deleted in the MG1655 Δ*ace*B Δ*gcl* strain by transduction. The MG1655 Δ*glc*B::Km is first constructed using the same method as previously described with the following oligonucleotides :
DglcBR (SEQ ID NO 9) 
cccagagccgtttacgcattgacgccaattttaaacgttttgtggatgaagaagttttaccgggaacagggctggacgcCATAT
GAATATCCTCCTTAG
 with
   - a region (lower case) homologous to the sequence (3121805-3121727) of the region of the gene *glc*B (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DglcBF (SEQ ID NO 10) 
cgcgtaaacgccaggcgtgtaataacggttcggtatagccgtttggctgtttcacgccgaggaagattaaatcgctggcTGTAG
GCTGGAGCTGCTTCG
 with
   - a region (lower case) homologous to the sequence (3119667-3119745) of the region of the gene *glc*B (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645).
The oligonucleotides DglcBF and DglcBR are used to amplify the kanamycin resistance cassette from the plasmid pKD4. The PCR product obtained is then introduced by electroporation into the strain MG1655 (pKD46). The kanamycin resistant transformants are then selected and the insertion of the resistance cassette is verified by a PCR analysis with the oligonucleotides glcBF and glcBR defined below. The strain retained is designated MG1655 Δ*glc*B::Km.
glcBR (SEQ ID NO 11) : gccagcaaatggcgagtgc (homologous to the sequence from 3122225 to 3122207).
glcBF (SEQ ID NO 12) : cgcagagtatcgttaagatgtcc (homologous to the sequence from 3119475 to 3119497).
To transfer the deletion Δ*glc*B::Km, the method of phage PI transduction is used. The preparation of the phage lysate of the strain MG1655 Δ*glc*B::Km is used for the transduction into strain MG1655 Δ*ace*B Δ*gcl.*

The kanamycin resistant transformants are then selected and the deletion of the gene Δ*glc*B::Km is verified by a PCR analysis with the previously defined oligonucleotides glcBF and glcBR. The strain retained is designated MG1655 Δ*ace*B Δ*gcl* Δ*glc*B::Km.

The kanamycin resistance cassette can then be eliminated. The plasmid pCP20 carrying FLP recombinase acting at the FRT sites of the kanamycin resistance cassette is then introduced into the recombinant sites by electroporation. After a series of cultures at 42°C, the loss of the kanamycin resistance cassette is verified by a PCR analysis with the same oligonucleotides as used previously (glcBF and glcBR). The strain retained is designated MG1655 Δ*ace*B Δ*gcl* Δ*glc*B.

### EXAMPLE 2

### Construction of strains with increased level of NADPH dependent glyoxylate reductase: MG1655 ΔaceB Δgcl ΔglcB (pME101-ycdW)

To boost the level of NADPH dependant glyoxylate reductase the *ycdW* gene is expressed from the plasmid pCL1920 (Lerner & Inouye, 1990, NAR 18, 15 p 4631) using the promoter Ptrc. For the expression from a low copy vector the plasmid pME101 is constructed as follows. The plasmid pCL1920 is PCR amplified using the oligonucleotides PME101F and PME101R and the BstZ17I-XmnI fragment from the vector PTRC99A harboring the *lac*I gene and the P_{trc} promoter is inserted into the amplified vector.
PME101F (SEQ ID NO 13) : Ccgacagtaagacgggtaagcctg
PME101R (SEQ ID NO 14) : Agcttagtaaagccctcgctag

The *ycd*W gene is PCR amplified from genomic DNA using the following oligonucleotides:
*Bsp*HI ycdW (SEQ ID NO 15): agctagctct**catgag**aataaatttcgcacaacgcttttcggg
*Sma*I ycdW (SEQ ID NO 16): gcatgcat**cccggg**tctctcctgtattcaattcccgcc

The PCR fragment is digested with *Bsp*HI and *Sma*I and cloned into the vector pME101 cut by the *Nco*I and *Sma*I restriction enzymes resulting in plasmid pME101-ycdW.
The pME101-ycdW plasmid is then introduced in the strain MG1655 Δ*ace*B Δ*gcl* Δ*glc*B*.*

### EXAMPLE 3

### Construction of strains with decreased consumption of glycolate : MG1655 ΔaceB Δgcl ΔglcDEFGB ΔaldA (pME101-ycdW)

The *glc*DEFGB genes are deleted in the MG1655 Δ*ace*B Δ*gcl* strain by transduction.

The MG1655 Δ*glc*DEFGB::Km is first constructed using the same method as previously described with the following oligonucleotides :
DglcDR (SEQ ID NO 17) 
gcgtcttgatggcgctttacccgatgtcgaccgcacatcggtactgatggcactgcgtgagcatgtccctggacttgagatccCA
TATGAATATCCTCCTTAG
 with
   - a region (lower case) homologous to the sequence (3126016-3125934) of the gene *glc*D (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),
DglcBF (SEQ ID NO 18) 
cgcgtaaacgccaggcgtgtaataacggttcggtatagccgtttggctgtttcacgccgaggaagattaaatcgctggcTGTAG
GCTGGAGCTGCTTCG
 with
   - a region (lower case) homologous to the sequence (3119667-3119745) of the region of the gene *glc*B (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645).
The oligonucleotides DglcDR and DglcBF are used to amplify the kanamycin resistance cassette from the plasmid pKD4. The PCR product obtained is then introduced by electroporation into the strain MG1655 (pKD46), in which the Red recombinase enzyme expressed permits the homologous recombination. The chloramphenicol resistant transformants are then selected and the insertion of the resistance cassette is verified by a PCR analysis with the oligonucleotides glcDR and glcBF defined below. The strain retained is designated MG1655 Δ*glc*DEFGB::Km
glcDR (SEQ ID NO 19) : ccaagacaaggtcacagagc (homologous to the sequence from 3126183 to 3126164).
glcBF (SEQ ID NO 20) : cgcagagtatcgttaagatgtcc (homologous to the sequence from 3119475 to 3119497).
To transfer the deletion Δ*glc*DEFGB::Km, the method of phage PI transduction is used.
The preparation of the phage lysate of the strain MG1655 Δ*glc*DEFGB::Km is used for the transduction into strain MG1655 Δ*ace*B Δ*gcl.*

The kanamycin resistant transformants are then selected and the deletion of the gene Δ*glc*DEFGB::Km is verified by a PCR analysis with the previously defined oligonucleotides glcBF and glcDR. The strain retained is designated MG1655 Δ*ace*B Δ*gcl* Δ*glc*DEFGB::Km.

Then, the *ald*A gene is deleted in the MG1655 Δ*ace*B Δ*gcl* Δ*glc*DEFGB::Km strain by transduction. The MG1655 *ald*A::Cm is first constructed using the same method as previously described with the following oligonucleotides :
AldA D r (SEQ ID NO 21) 
ttaagactgtaaataaaccacctgggtctgcagatattcatgcaagccatgtttaccatctgcgccgccaataccggatttCATAT
GAATATCCTCCTTAG
 with
   - a region (lower case) homologous to the sequence (1487615 to 1487695) of the gene *ald*A (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
AldA D f (SEQ ID NO 22) 
atgtcagtacccgttcaacatcctatgtatatcgatggacagtttgttacctggcgtggagacgcatggattgatgtggtaGTGTA
GGCTGGAGCTGCTTCG
 with
   - a region (lower case) homologous to the sequence (1486256 to 1486336) of the gene *ald*A (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645).
The oligonucleotides aldAF and aldAR are used to amplify the chloramphenicol resistance cassette from the plasmid pKD3. The PCR product obtained is then introduced by electroporation into the strain MG1655 (pKD46), in which the Red recombinase enzyme expressed permits the homologous recombination. The kanamycin resistant transformants are then selected and the insertion of the resistance cassette is verified by a PCR analysis with the oligonucleotides YdcFCf and gapCCR defined below. The strain retained is designated MG1655 Δ*ald*A::Cm*.*

YdcFCf (SEQ ID NO 23) : tgcagcggcgcacgatggcgacgttccgccg (homologous to the sequence from 1485722 to 1485752).
gapCCR (SEQ ID NO 24) : cacgatgacgaccattcatgcctatactggc (homologous to the sequence from 1488195 to 1488225).
To transfer the deletion Δ*ald*A::Cm, the method of phage PI transduction is used. The preparation of the phage lysate of the strain MG1655 Δ*ald*A::Cm is used for the transduction into strain MG1655 Δ*ace*B Δ*gcl* Δ*glc*DEFGB::Km.

The kanamycin resistant transformants are then selected and the deletion of the gene Δ*ald*A::Cm is verified by a PCR analysis with the previously defined oligonucleotides YdcFCf and gapCCR. The strain retained is designated MG1655 Δ*ace*B Δ*gcl* Δ*glc*DEFGB::Km Δ*ald*A::Cm*.*

The kanamycin and chloramphenicol resistance cassettes can then be eliminated. The plasmid pCP20 carrying FLP recombinase acting at the FRT sites of the kanamycin and the chloramphenicol resistance cassettes is then introduced into the recombinant sites by electroporation. After a series of cultures at 42°C, the loss of the kanamycin and chloramphenicol resistance cassettes is verified by a PCR analysis with the same oligonucleotides as used previously (glcBF / glcDR and YdcFCf / gapCCR). The strain retained is designated MG1655 Δ*ace*B Δ*gcl* Δ*glc*DEFGB Δ*ald*A*.*
The pME101-ycdW plasmid is then introduced in the strain MG1655 Δ*ace*B Δ*gcl* Δ*glc*DEFGB Δ*ald*A*.*

### EXAMPLE 4

### Construction of strains with increased flux in the glyoxylate pathway : MG1655 ΔaceB Δgcl ΔglcDEFGB ΔaldA ΔiclR (pME101-ycdW)

The *icl*R gene deletion is introduced in the MG1655 Δ*ace*B Δ*gcl* Δ*glc*DEFGB Δ*ald*A using the same strategy as previously described with the following oligonucleotides :
DiclF (SEQ ID NO 25) 
CgcacccattcccgcgaaacgcggcagaaaacccgccgttgccaccgcaccagcgactggacaggttcagtctttaacgcgT
GTAGGCTGGAGCTGCTTCG
 with
   - a region (lower case) homologous to the sequence (4221202-4221120) of the gene *icl*R (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645),
DiclR (SEQ ID NO 26) 
gcgcattccaccgtacgccagcgtcacttccttcgccgctttaatcaccatcgcgccaaactcggtcacgcggtcatcggCATA
TGAATATCCTCCTTAG
 with
   - a region (lower case) homologous to the sequence (4220386-4220465) of the gene *icl*R (reference sequence on the website http://genolist.pasteur.fr/Colibri/),
   - a region (upper case) for the amplification of the kanamycin resistance cassette (reference sequence in Datsenko, K.A. & Wanner, B.L., 2000, PNAS, 97: 6640-6645).
The oligonucleotides DiclF and DiclR are used to amplify the kanamycin resistance cassette from the plasmid pKD4. The PCR product obtained is then introduced by electroporation into the strain MG1655 Δ*ace*B Δ*gcl* Δ*glc*DEFGB Δ*ald*A (pKD46). The kanamycin resistant transformants are then selected and the insertion of the resistance cassette is verified by a PCR analysis with the oligonucleotides iclF and iclR defined below. The strain retained is designated MG1655 Δ*ace*B Δ*gcl* Δ*glc*DEFGB Δ*ald*A Δ*icl*R::Km
IclF (SEQ ID NO 27) : cctttgaggtcgcatggccagtcggc (homologous to the sequence from 4221558 to 4221533).
iclR (SEQ ID NO 28) : gctttttaatagaggcgtcgccagctccttgcc (homologous to the sequence from 4219917 to 4219949).

The kanamycin resistance cassette can then be eliminated. The plasmid pCP20 carrying FLP recombinase acting at the FRT sites of the kanamycin resistance cassette is then introduced into the recombinant sites by electroporation. After a series of cultures at 42°C, the loss of the kanamycin resistance cassette is verified by a PCR analysis with the same oligonucleotides as used previously (iclF and iclR). The strain retained is designated MG1655 Δ*ace*B Δ*gcl* Δ*glc*DEFGB Δ*ald*A Δ*icl*R.
The pME101-ycdW plasmid is then introduced in the strain MG1655 Δ*ace*B Δ*gcl* Δ*glc*DEFGB Δ*ald*A Δ*icl*R*.*

### EXAMPLE 5

### CONSTRUCTION OF STRAINS WITH INCREASED NADPH AVAILABILITY: MG1655 ΔaceB Δgcl ΔiclR ΔglcDEFGB ΔaldA Δedd-eda (pME101-ycdW)

The *edd-eda* genes are deleted in the MG1655 Δ*ace*B Δ*gcl* Δ*glc*B Δ*glc*DEF Δ*ald*A Δ*icl*R strain by transduction.
The strain MG1655 Δ*edd-eda*::Cm is first constructed using the same method as previously described with the following oligonucleotides :
DeddF (SEQ ID NO 29) 
CgcgcgagactcgctctgcttatctcgcccggatagaacaagcgaaaacttcgaccgttcatcgttcgcagttggcatgcggTG
TAGGCTGGAGCTGCTTCG
 with
   - a region (lower case) homologous to the sequence (1932582-1932500) of the region of the genes *edd-eda* (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),
DedaR (SEQ ID NO 30) 
gcttagcgccttctacagcttcacgcgccagcttagtaatgcggtcgtaatcgcccgcttccagcgcatctgccggaaccCATA
TGAATATCCTCCTTAG
 with
   - a region (lower case) homologous to the sequence (1930144-1930223) of the region of the genes *edd-eda* (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),
The oligonucleotides DeddF and DedaR are used to amplify the chloramphenicol resistance cassette from the plasmid pKD3. The PCR product obtained is then introduced by electroporation into the strain MG1655 (pKD46). The chloramphenicol resistant transformants are then selected and the insertion of the resistance cassette is verified by a PCR analysis with the oligonucleotides eddF and edaR defined below. The strain retained is designated MG1655 Δ*edd-eda*::Cm.
eddF (SEQ ID NO 31) : Gggtagactccattactgaggcgtgggcg (homologous to the sequence from 1932996 to 1932968).
edaR (SEQ ID NO 32) : ccacatgataccgggatggtgacg (homologous to the sequence from 1929754 to 1929777).

To transfer the deletion Δ*edd-eda*::Cm, the method of phage PI transduction as previously described is used. The preparation of the phage lysate of the strain MG1655 Δ*edd-eda*::Cm was used for the transduction into strain MG1655 Δ*ace*B Δ*gcl* Δ*glc*DEFGB Δ*ald*A Δ*icl*R.

The chloramphenicol resistant transformants are then selected and the deletion of the gene Δ*edd-eda*::Cm is verified by a PCR analysis with the oligonucleotides eddF and edaR. The strain retained is designated MG1655 Δ*ace*B Δ*gcl* Δ*glc*B Δ*glc*DEF Δ*ald*A Δ*icl*R Δ*edd-eda*::Cm.

The chloramphenicol resistance cassette can then be eliminated. The plasmid pCP20 carrying FLP recombinase acting at the FRT sites of the chloramphenicol resistance cassette is then introduced into the recombinant sites by electroporation. After a series of cultures at 42°C, the loss of the chloramphenicol resistance cassette is verified by a PCR analysis with the same oligonucleotides as used previously (eddF and edaR). The strain retained is designated MG1655 Δ*ace*B Δ*gcl* Δ*glc*DEFGB Δ*ald*A Δ*icl*R Δ*edd-eda.*

The pME101-ycdW plasmid is then introduced in the strain MG1655 Δ*ace*B Δ*gcl* Δ*glc*DEFGB Δ*ald*A Δ*icl*R Δ*edd-eda.*

### EXAMPLE 6

### CONSTRUCTION OF STRAINS WITH INCREASED NADPH AVAILABILITY: MG1655 ΔaceB Δgcl ΔiclR ΔglcDEFGB ΔaldA Δpgi::Cm Δedd-eda (pME101-ycdW)

The *pgi* gene deletion is introduced in the MG1655 Δ*ace*B Δ*gcl* Δ*glc*B Δ*glc*DEF Δ*ald*A Δ*icl*R Δ*edd-eda* using the same strategy as previously described with the following oligonucleotides:
DpgiF (SEQ ID NO 33) 
ccaacgcagaccgctgcctggcaggcactacagaaacacttcgatgaaatgaaagacgttacgatcgccgatctttttgcTGTA
GGCTGGAGCTGCTTCG
 with
   - a region (lower case) homologous to the sequence (4231352-4231432) of the gene *pgi* (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),
DpgiR (SEQ ID NO 34) 
gcgccacgctttatagcggttaatcagaccattggtcgagctatcgtggctgctgatttctttatcatctttcagctctgCATATG
AATATCCTCCTTAG
 with
   - a region (lower case) homologous to the sequence (4232980-4232901) of the gene *pgi* (reference sequence on the website http://genolist.pasteur.fr/Colibri/),
   - a region (upper case) for the amplification of the chloramphenicol resistance cassette (reference sequence in Datsenko, K.A. & Wanner, B.L., 2000, PNAS, 97: 6640-6645),

The oligonucleotides DpgiF and DpgiR are used to amplify the chloramphenicol resistance cassette from the plasmid pKD3. The PCR product obtained is then introduced by electroporation into the strain MG1655 Δ*ace*B Δ*gcl* Δ*glc*B Δ*glc*DEF Δ*ald*A Δ*icl*R Δ*edd-eda* (pKD46). The chloramphenicol resistant transformants are then selected and the insertion of the resistance cassette is verified by a PCR analysis with the oligonucleotides pgiF and pgiR defined below. The strain retained is designated MG1655 Δ*ace*B Δ*gcl* Δ*glc*B Δ*glc*DEF Δ*ald*A Δ*icl*R Δ*edd-eda* Δ*pgi*::Cm
pgiF (SEQ ID NO 35) : gcggggcggttgtcaacgatggggtcatgc (homologous to the sequence from 4231138 to 4231167).
pgiR (SEQ ID NO 36) : cggtatgatttccgttaaattacagacaag (homologous to the sequence from 4233220 to 4233191).

The pME101-ycdW plasmid is then introduced in the strain MG1655 Δ*ace*B Δ*gcl* Δ*glc*DEFGB Δ*ald*A Δ*icl*R Δ*edd-eda* Δ*pgi*::Cm.

### EXAMPLE 7

### CONSTRUCTION OF STRAINS WITH INCREASED NADPH AVAILABILITY: MG1655 ΔaceB Δgcl ΔiclR ΔglcDEFGB ΔaldA Δpgi Δedd-eda::Cm ΔudhA::Km (pME101-ycdW)

The *udhA* gene deletion is introduced in the MG1655 Δ*ace*B Δ*gcl* Δ*glc*DEFGB Δ*ald*A Δ*icl*R Δ*pgi*::Cm Δ*edd-eda* using the same strategy as previously described with the following oligonucleotides :
DudhAF (SEQ ID NO 37) 
CCCAGAATCTCTTTTGTTTCCCGATGGAACAAAATTTTCAGCGTGCCCACGTTC
ATGCCGACGATTTGTGCGCGTGCCAGTGTAGGCTGGAGCTGCTTCG
 with
   - a region (boldface letters) homologous to the sequence (4157588-4157667) of the gene *udh*A (reference sequence on the website http://genolist.pasteur.fr/Colibri/),
   - a region (underlined letters) for the amplification of the kanamycin resistance cassette (reference sequence in Datsenko, K.A. & Wanner, B.L., 2000, PNAS, 97: 6640-6645),
DudhAR (SEQ ID NO 38) 
GGTGCGCGCGTCGCAGTTATCGAGCGTTATCAAAATGTTGGCGGCGGTTGCAC
CCACTGGGGCACCATCCCGTCGAAAGCCATATGAATATCCTCCTTAG
 with
   - a region (boldface letters) homologous to the sequence (4158729-4158650) of the gene *udh*A (reference sequence on the website http://genolist.pasteur.fr/Colibri/),
   - a region (underlined letters) for the amplification of the kanamycin resistance cassette (reference sequence in Datsenko, K.A. & Wanner, B.L., 2000, PNAS, 97: 6640-6645),

The oligonucleotides DudhAF and DudhAR are used to amplify the kanamycin resistance cassette from the plasmid pKD4. The PCR product obtained is then introduced by electroporation into the strain MG1655 Δ*ace*B Δ*gcl* Δ*glc*DEFGB Δ*ald*A Δ*icl*R Δ*pgi*::Cm Δ*edd-eda* (pKD46). The kanamycin resistant transformants are then selected and the insertion of the resistance cassette is verified by a PCR analysis with the oligonucleotides udhAF and udhAR defined below. The strain retained is designated MG1655 Δ*ace*B Δ*gcl* Δ*glc*DEFGB Δ*ald*A Δ*icl*R Δ*pgi*::Cm Δ*edd-eda* Δ*udh*A::Km
udhAF (SEQ ID NO 39) : (homologous to the sequence from 4157088 to 4157108). GATGCTGGAAGATGGTCACT
udhAR (SEQ ID NO 40) : (homologous to the sequence from 4159070 to 4159052). gtgaatgaacggtaacgc

The pME101-ycdW plasmid is then introduced in the strain MG1655 Δ*ace*B Δ*gcl* Δ*glc*DEFGB Δ*ald*A Δ*icl*R Δ*pgi*::Cm Δ*edd-eda* Δ*udh*A::Km.

### EXAMPLE 8

### FERMENTATION OF GLYCOLIC ACID PRODUCING STRAINS IN ERLENMEYER FLASKS

Performances of strains were initially assessed in 250 ml baffled Erlenmeyer flask cultures using modified M9 medium (Anderson, 1946, Proc. Natl. Acad. Sci. USA 32:120-128) that was supplemented with 40 g/l MOPS and 10 g/l glucose and adjusted at pH 6.8. Spectinomycin was added if necessary at a concentration of 50 mg/l and 100 µm IPTG was also added for induction of the expression vector, if present. An overnight preculture was used to inoculate a 50 ml culture to an OD_{600 nm} of about 0.3. The cultures were kept on a shaker at 30°C and 400 rpm until the glucose in the culture medium was exhausted. At the end of the culture, glucose and glycolic acid were analyzed by HPLC using a Biorad HPX 97H column for the separation and a refractometer for the detection.

Comparison of the performances of the different strains is given in table below (each value is the mean of n repetitions). The strain described in example 1 did not show any production of glycolic acid.

| **Strain from example n°** | 2 | 3 | 4 | 5 | 6 | 7 |
|---|---|---|---|---|---|---|
| **Genotype (*E. coli* MG1655)** | Δ*ace*B Δ*gcl* Δ*glc*B (pME101-*ycd*W) | Δ*ace*B Δ*gcl* Δ*glc*DEF GB Δ*ald*A (pME101-*ycd*W) | Δ*ace*B Δ*gcl* Δ*glc*DEF GB Δ*ald*A Δ*icl*R (pME101-*ycd*W) | Δ*ace*B Δ*gcl* Δ*glc*DEF GB Δ*ald*A Δ*icl*R Δ*edd-eda* (pME101-*ycd*W) | Δ*ace*B Δ*gcl* Δ*glc*DEF GB Δ*ald*A Δ*icl*R Δ*edd-eda* Δ*pgi* (pME101-*ycd*W) | Δ*ace*B Δ*gcl* Δ*glc*DEF GB Δ*ald*A Δ*icl*R Δ*edd-eda* Δ*pgi* Δ*udh*A (pME101-*ycd*W) |
| **Glycolic acid production (g/l)** | 0.28 (n = 3) | 0.28 (n = 8) | 0.65 (n = 8) | 1.73 (n = 8) | 2.75 (n = 6) | 2.33 (n = 4) |
| **Yield (g glycolic acid / g glucose)** | 0.03 (n = 3) | 0.03 (n = 8) | 0.07 (n = 8) | 0.17 (n = 8) | 0.29 (n = 6) | 0.25 (n = 4) |

Strains described in example 6 and example 7 are the best producers of glycolic acid with titers higher than 2 g/l and yields higher than 0.2 g/g.

### EXAMPLE 9

### FERMENTATION OF GLYCOLIC ACID PRODUCING STRAINS IN FED BATCH FERMENTOR

The strains described in example 6 and in example 7 were assessed under production conditions in a 600 ml fermentor using a fed batch protocol.

A first preculture in tubes was carried out in LB medium supplemented with 2,5 g/l of glucose at 30°C followed by a second preculture in 500 ml Erlenmeyer flask filled with 50 ml of synthetic medium supplemented with 40 g/l of MOPS and 10 g/l of glucose (the same medium used for flask cultures) at 30°C. This second preculture was used for inoculation of the fermentor.

The fermentor filled with 200 ml of synthetic medium supplemented with 40 g/l of glucose, 50 mg/l of spectinomycin and 100 µM IPTG was inoculated at an initial optical density of about 2. The culture was carried out at 30°C with agitation and aeration adjusted to maintain the dissolved oxygen above 30% saturation. The pH was adjusted at 6.8 with base addition. The culture was conducted in a batch mode until exhaustion of glucose. At that time, a solution of 500 g/l glucose supplemented with magnesium sulfate, oligo-elements, spectinomycin and IPTG was added to restore a concentration of 40 g/l of glucose in the medium. Other additions were done each time glucose became exhausted again.
Routinely, strain described in example 7 gave better production performance than strain described in example 6 in fermentors (yield from glucose 0.22 g/g versus 0.15 g/g).

A representative time-course of fermentation for production of glycolic acid using strain of example 7 is given below.

| **Time (h)** | **OD_{600 nm} (AU)** | **Glucose (g/l)** | **Glycolic acid (g/l)** |
|---|---|---|---|
| 0 | 2.0 | 35.45 | 0.11 |
| 16 | 3.7 | 34.70 | 0.57 |
| 20 | 5.1 | 33.25 | 1.14 |
| 25 | 6.7 | 31.24 | 1.81 |
| 39 | 14.8 | 20.55 | 4.75 |
| 44 | 20.3 | 12.24 | 7.02 |
| 49 | 27.9 | 2.48 | 9.44 |
| 64 | 53.9 | 7.94 | 18.80 |
| 70 | 62.8 | 33.97 | 21.76 |
| 73 | 67.8 | 24.54 | 23.54 |
| 87 | 84.0 | 36.60 | 28.70 |
| 93 | 89.6 | 25.61 | 31.33 |

The final titre obtained was 31 g/l glycolic acid with a yield on glucose of 0.22 g/g.

## Claims

1. A method for the fermentative production of glycolic acid by culturing a recombinant microorganism modified for converting a source of carbon to glycolic acid, in an appropriate culture medium comprising a fermentable source of carbon capable of being metabolized by the microorganism, said method comprising the steps of:
a) Fermentation of the microorganism to produce glycolic acid by converting the source of carbon into glycolic acid,
b) Concentration of the glycolic acid in the microorganism or in the medium and
c) Recovery of glycolic acid from the fermentation broth and/or the biomass optionally remaining in portions or in the total amount (0-100%) in the end product, wherein the fermentable source of carbon is a sugar selected among monosaccharides, disaccharides, trisaccharides, oligosaccharides and polysaccharides,
and wherein the microorganism is modified to attenuate the conversion of glyoxylate to products other than glycolate, with the attenuation of the expression of at least one gene, selected among the following genes involved in glyoxylate metabolism:
• *ace*B encoding malate synthase
• *glc*B encoding the second malate synthase
• *gcl* encoding glyoxylate carboligase
• *eda* encoding 2-keto-3-deoxygluconate 6-phosphate aldolase.

2. A method as claimed in claim 1 wherein glycolate is isolated through a step of polymerization to at least glycolate dimers.

3. A method as claimed in claim 2 wherein glycolate is recovered by depolymerization from glycolate dimers, oligomers and/or polymers.

4. A method as claimed in claim 1 wherein the microorganism contains at least one gene encoding a polypeptide catalyzing the conversion of glyoxylate to glycolate.

5. A method as claimed in claim 4 wherein the gene encodes a NADPH dependent glyoxylate reductase.

6. A method as claimed in claim 5 in which the gene encoding a polypeptide with NADPH dependent glyoxylate reductase activity is endogenous.

7. A method as claimed in any one of claims 4 to 6 wherein the expression of said gene is increased.

8. A method as claimed in any one of claims 5 to 7 wherein the gene encoding a polypeptide with NADPH dependent glyoxylate reductase activity is selected among *ycd*W and *yia*E.

9. A method as claimed in any one of claims 1 to 8 wherein the microorganism is modified in such a way that it is unable to substantially metabolize glycolate, the expression of at least one gene, selected among the following genes involved in glycolate metabolism, being attenuated in the microorganism:
• *glc*DEF encoding glycolate oxidase
• *aldA* encoding glycoaldehyde dehydrogenase.

10. A method as claimed in any one of claims 1 to 9 wherein the microorganism is transformed to increase the glyoxylate pathway flux, by:
- attenuating the activity of the enzyme isocitrate dehydrogenase, or
- attenuating the expression of at least one of the following genes:
• *pta* encoding phospho-transacetylase
• *ack* encoding acetate kinase
• *pox*B encoding pyruvate oxidase, or
- increasing the activity of aceA.

11. A method as claimed in claim 10 wherein the expression of *ace*A is increased by the attenuation of the expression of the genes *icl*R *or fad*R*.*

12. A method as claimed in claim 10 wherein the expression of *ace*A is increased by introducing an artificial promoter upstream of the gene *ace*A*.*

13. A method as claimed in any one of claims 1 to 12 wherein the availability of NADPH is increased, by attenuating the expression of at least one gene, selected among the following:
• *pgi* encoding the glucose-6-phosphate isomerase
• *udh*A encoding the soluble transhydrogenase
• *edd* encoding phosphogluconate dehydratase.

14. A method as claimed in any one of claims 1 to 13, wherein the carbon source is at least one of the following: glucose, sucrose, mono- or oligosaccharides, or starch.

15. A recombinant microorganism as defined in any one of claims 1 to 14.

16. A microorganism as claimed in claim 15 that is selected among the group consisting of *E. coli, C. glutamicum* or *S. cerevisiae.*

## Patentansprüche

1. Verfahren zur fermentativen Herstellung von Glykolsäure durch Kultivieren eines rekombinanten Mikroorganismus, modifiziert zur Umwandlung einer Kohlenstoffquelle in Glykolsäure in einem geeigneten Kulturmedium, umfassend eine fermentierbare Kohlenstoffquelle, die vom Mikroorganismus metabolisiert werden kann, wobei das Verfahren die folgenden Schritte umfasst:
a) Fermentieren des Mikroorganismus, um Glykolsäure durch Umwandeln der Kohlenstoffquelle in Glykolsäure herzustellen,
b) Konzentrieren der Glykolsäure im Mikroorganismus oder im Medium, und
c) Wiedergewinnen von Glykolsäure aus der Fermentationsbrühe und/oder der Biomasse, die optional in Abschnitten oder in der Gesamtmenge (0-100 %) im Endprodukt verbleibt,
wobei die fermentierbare Kohlenstoffquelle Zucker ist, ausgewählt aus Monosacchariden, Disacchariden, Trisacchariden, Oligosacchariden und Polysacchariden,
und wobei der Mikroorganismus modifiziert ist, um die Umwandlung von Glyoxylat in Produkte außer Glycolat zu dämpfen, mit der Dämpfung der Expression von mindestens einem Gen, ausgewählt aus den folgenden Genen, die am Glyoxylatmetabolismus beteiligt sind:
• *ace*B codierend für Malatsynthase
• *glc*B codierend für die zweite Malatsynthase
• *gcl* codierend für Glyoxylatcarboligase
• *eda* codierend für 2-Keto-3-deoxygluconat 6-phosphataldolase.

2. Verfahren nach Anspruch 1, wobei Glycolat durch einen Schritt der Polymerisierung zu mindestens Glycolatdimeren isoliert wird.

3. Verfahren nach Anspruch 2, wobei Glycolat durch Depolymerisierung aus Glykolatdimeren, Oligomeren und/oder Polymeren wiedergewonnen wird.

4. Verfahren nach Anspruch 1, wobei der Mikroorganismus mindestens ein Gen enthält, das für ein Polypeptid codiert, das die Umwandlung von Glyoxylat zu Glycolat katalysiert.

5. Verfahren nach Anspruch 4, wobei das Gen für eine NADPH-abhängige Glyoxylatreductase codiert.

6. Verfahren nach Anspruch 5, wobei das Gen, codierend für ein Polypeptid mit NADPH-abhängiger Glyoxylatreductase-Aktivität, endogen ist.

7. Verfahren nach einem der Ansprüche 4 bis 6, wobei die Expression des Gens erhöht ist.

8. Verfahren nach einem der Ansprüche 5 bis 7, wobei das Gen, codierend für ein Polypeptid mit NADPH-abhängiger Glyoxylatreductase-Aktivität, ausgewählt ist aus *ycd*W und *yia*E*.*

9. Verfahren nach einem der Ansprüche 1 bis 8, wobei der Mikroorganismus derart modifiziert wird, das er nicht dazu in der Lage ist, im Wesentlichen Glycolat zu metabolisieren, wobei die Expression von mindestens einem Gen, ausgewählt aus den folgenden Genen, die am Glycolatmetabolismus beteiligt sind, im Mikroorganismus gedämpft wird:
• *glc*DEF codierend für Glycolatoxidase
• *aldA* codierend für Glycoaldehyddehydrogenase.

10. Verfahren nach einem der Ansprüche 1 bis 9, wobei der Mikroorganismus umgewandelt wird, um den Glyoxylat-Durchfluss zu erhöhen durch:
- Dämpfen der Aktivität des Enzyms Isocitrat-Dehydrogenase, oder
- Dämpfen der Expression von mindestens einem der folgenden Gene:
• *pta* codierend für Phospho-transacetylase
• *ack* codierend für Acetatkinase
• *pox*B codierend für Pyruvatoxidase, oder
- Erhöhen der Aktivität von aceA.

11. Verfahren nach Anspruch 10, wobei die Expression von *ace*A durch die Dämpfung der Expression der Gene *icl*R oder *fad*R erhöht wird.

12. Verfahren nach Anspruch 10, wobei die Expression von *ace*A durch die Einführung eines künstlichen Promotors vorgelagert vom Gen *ace*A erhöht wird.

13. Verfahren nach einem der Ansprüche 1 bis 12, wobei die Verfügbarkeit von NADPH durch Dämpfen der Expression von mindestens einem Gen erhöht wird, ausgewählt aus den Folgenden:
• *pgi* codierend für Glucose-6-phosphatisomerase
• *udh*A codierend für die lösliche Transhydrogenase
• *edd* codierend für Phosphogluconatdehydratase.

14. Verfahren nach einem der Ansprüche 1 bis 13, wobei die Kohlenstoffquelle mindestens eines der Folgenden ist: Glucose, Sucrose, Mono- oder Oligosaccharide oder Stärke.

15. Rekombinanter Mikroorganismus wie in einem der Ansprüche 1 bis 14 definiert.

16. Mikroorganismus nach Anspruch 15, der ausgewählt ist aus der Gruppe, bestehend aus *E*. *coli, C. glutamicum* oder *S. cerevisiae.*

## Revendications

1. Méthode de production par fermentation d'acide glycolique par la culture d'un micro-organisme recombinant modifié pour convertir une source de carbone en acide glycolique, dans un milieu de culture approprié comprenant une source fermentable de carbone pouvant être métabolisée par le micro-organisme, ladite méthode comprenant les étapes de :
a) la fermentation du micro-organisme pour produire l'acide glycolique par conversion de la source de carbone en acide glycolique,
b) la concentration de l'acide glycolique dans le micro-organisme ou dans le milieu et
c) la récupération de l'acide glycolique à partir du bouillon de fermentation et/ou de la biomasse restant éventuellement dans des parties ou dans la quantité totale (0 à 100 %) du produit final,
dans laquelle la source fermentable de carbone est un sucre sélectionné parmi des monosaccharides, des disaccharides, des trisaccharides, des oligosaccharides et des polysaccharides,
et dans laquelle le micro-organisme est modifié pour atténuer la conversion du glyoxylate en des produits autres que le glycolate, avec l'atténuation de l'expression d'au moins un gène, sélectionné parmi les gènes suivants impliqués dans le métabolisme du glyoxylate :
• *aceB* codant pour la malate synthase
• *glc*B codant pour la seconde malate synthase
• *gcl* codant pour la glyoxylate carboligase
• *eda* codant pour la 2-céto-3-désoxygluconate-6-phosphate aldolase.

2. Méthode selon la revendication 1 dans laquelle le glycolate est isolé par l'intermédiaire d'une étape de polymérisation en au moins des dimères de glycolate.

3. Méthode selon la revendication 2 dans laquelle le glycolate est récupéré par dépolymérisation à partir des dimères, des oligomères et/ou des polymères de glycolate.

4. Méthode selon la revendication 1 dans laquelle le micro-organisme contient au moins un gène codant pour un polypeptide catalysant la conversion de glyoxylate en glycolate.

5. Méthode selon la revendication 4 dans laquelle le gène code pour une glyoxylate réductase NADPH-dépendante.

6. Méthode selon la revendication 5 dans laquelle le gène codant pour un polypeptide doté d'une activité glyoxylate réductase NADPH-dépendante est endogène.

7. Méthode selon l'une quelconque des revendications 4 à 6 dans laquelle l'expression dudit gène est augmentée.

8. Méthode selon l'une quelconque des revendications 5 à 7 dans laquelle le gène codant pour un polypeptide doté d'une activité glyoxylate réductase NADPH-dépendante est sélectionné parmi *ycd*W et *yia*E*.*

9. Méthode selon l'une quelconque des revendications 1 à 8 dans laquelle le micro-organisme est modifié de telle façon qu'il est incapable de métaboliser sensiblement le glycolate, l'expression d'au moins un gène, sélectionné parmi les gènes suivants impliqués dans le métabolisme du glycolate, étant atténuée dans le micro-organisme :
• *glc*DEF codant pour la glycolate oxydase
• *ald*A codant pour la glycoaldéhyde déshydrogénase.

10. Méthode selon l'une quelconque des revendications 1 à 9 dans laquelle le micro-organisme est transformé pour augmenter le flux de la voie du glyoxylate, par :
- l'atténuation de l'activité de l'enzyme isocitrate déshydrogénase, ou
- l'atténuation de l'expression d'au moins l'un des gènes suivants :
• *pta* codant pour la phospho-transacétylase
• *ack* codant pour l'acétate kinase
• *pox*B codant pour la pyruvate oxydase, ou
- l'augmentation de l'activité de *ace*A*.*

11. Méthode selon la revendication 10 dans laquelle l'expression de *ace*A est augmentée par l'atténuation de l'expression des gènes *icl*R ou *fad*R*.*

12. Méthode selon la revendication 10 dans laquelle l'expression de *ace*A est augmentée par l'introduction d'un promoteur artificiel en amont du gène *ace*A*.*

13. Méthode selon l'une quelconque des revendications 1 à 12 dans laquelle la disponibilité du NADPH est augmentée, par l'atténuation de l'expression d'au moins un gène, sélectionné parmi les suivants :
• *pgi* codant pour la glucose-6-phosphate isomérase
• *udhA* codant pour la transhydrogénase soluble
• *edd* codant pour la phosphogluconate déshydratase.

14. Méthode selon l'une quelconque des revendications 1 à 13, dans laquelle la source de carbone est au moins l'une des suivantes : le glucose, le saccharose, des mono- ou oligosaccharides, ou l'amidon.

15. Micro-organisme recombinant tel que défini dans l'une quelconque des revendications 1 à 14.

16. Micro-organisme selon la revendication 15 qui est sélectionné dans le groupe consistant en *E*. *coli, C. glutamicum* ou *S. cerevisiae.*
